# EUROPEAN PATENT SPECIFICATION

(11) **EP 2 636 679 B1**
(45) Date of publication and mention of the grant of the patent: **20.05.2015**
(21) Application number: 13153899.3
(22) Date of filing: 13.09.2005
(51) Int. Cl.: C07K 7/06

(54) **HLA-binding peptide, and DNA fragment and recombinant vector coding for said HLA-binding peptide**
HLA-bindendes Peptid und DNA-Fragment und rekombinanter Vektor zur Kodierung dieses HLA-bindenden Peptids
Peptide de liaison HLA, fragment d'ADN et vecteur de recombinaison codant pour ce peptide de liaison HLA

(30) Priority: 17.09.2004 JP 2004272385; 23.02.2005 JP 2005046459
(43) Date of publication of application: 11.09.2013
(62) Divisional of application: 05783164.6
(73) Proprietor: NEC Corporation, Tokyo 108-8001 (JP); Kochi University, Kochi-shi Kochi 7808520 (JP)
(72) Inventor: Miyakawa, Tomoya, Tokyo 108-8001 (JP); Udaka, Keiko, Kohasu, Kochi 783-8505 (JP)
(74) Representative: Vossius & Partner Patentanwälte Rechtsanwälte mbB

(56) References cited:
- WO-A1-02/28414

## Description

### TECHNICAL FIELD

The invention relates to an HLA-binding peptide consisting of SEQ ID NO: 38 which binds to a human HLA-A2 molecule.

### BACKGROUND ART

When a cancer antigen that is specific to a cancer cell is present on the surface of the cancer cell, there are times when an innate immune reaction as a result of the cancer cell being recognized as a substance foreign to oneself proceeds, and a specific immune response is subsequently induced to thus cause a reaction to eliminate the cancer cell.

When a specific immune response is induced, cancer cell-derived fragments and the like in body fluids are eliminated by neutralizing antibodies, and the cancer cells themselves are eliminated by cytotoxic T lymphocytes (CTLs). That is, the CTL specifically recognizes a cancer antigen (CTL epitope) consisting of 8 to 11 amino acids presented in an HLA class I molecule on the surface of a cancer cell, and eliminates the cancer by damaging the cancer cell. Therefore, it is critical to identify such a cancer-specific CTL epitope in order to develop a therapeutic vaccine for the cancer.

A technique of this kind is known from Patent Publication 1. Patent Publication 1 states that an oligopeptide formed from a specific amino acid sequence has the property of binding to an HLA.
[Patent Publication 1] Japanese Patent Application Laid-open No. H8-151396 (1996)

### DISCLOSURE OF THE INVENTION

However, the conventional technique described in the above-mentioned publication has room for improvement with regard to the following points.

Firstly, it is unclear whether or not the HLA-binding peptide of the above-mentioned publication binds to an HLA molecule effectively, and there is still room for improvement in terms of the HLA-binding properties.

Secondly, it is stated that the HLA-binding peptide of the above-mentioned publication has the property of binding to HLA-DQ4. However, it is unclear whether or not it binds to an HLA-A2 molecule (product of the HLA-A*0201 gene and the like), which is often seen in European and American people, and an HLA-A24 molecule (product of the HLA-A*2402 gene and the like), which is often seen in Japanese people.

The invention has been accomplished under the above-mentioned circumstances, and it is an object thereof to provide an HLA-binding peptide that exhibits high-affinity binding to a specific type of HLA molecule.

According to the present disclosure, there is provided an HLA-binding peptide binding to an HLA-A type molecule, the HLA-binding peptide containing at least one type of amino acid sequence selected from the group consisting of SEQ ID NOS: 1 to 60, and consisting of not less than 8 and not more than 11 amino acid residues.

Furthermore, according to the invention, there is provided the HLA-binding peptide consisting of the amino acid sequence of SEQ ID NO: 38.

Moreover, according to the present disclosure, there is provided an HLA-binding peptide binding to an HLA-A type molecule, the HLA-binding peptide containing an amino acid sequence formed by deletion, substitution, or addition of one or two amino acid residues of the amino acid sequence contained in the above-mentioned HLA-binding peptide, and consisting of not less than 8 and not more than 11 amino acid residues.

In this way, the construct containing an amino acid sequence formed by deletion, substitution, or addition of one or a few amino acid residues of a specific amino acid sequence that has the property of binding to an HLA-A type molecule can also exhibit a similar effect to that of the above-mentioned HLA-binding peptide.

Furthermore, according to the present disclosure, there isdescribeda DNA segment containing a DNA sequence coding for the above-mentioned HLA-binding peptide.

Moreover, according to the present disclosure, there is described a recombinant vector containing a DNA sequence coding for the above-mentioned HLA-binding peptide.

Furthermore, according to the present disclosure, there is described an HLA-binding peptide precursor changing within a mammalian body into the above-mentioned HLA-binding peptide.

Constructs of the present disclosure are explained above, but any combination of these constructs is also effective as an embodiment herein.

Furthermore, conversion of the expression of the present disclosureinto another category is also effective as an embodiment of the present disclosure.

In accordance with the present disclosure, since it includes a specific amino acid sequence, an HLA-binding peptide that has excellent properties in binding to an HLA-A type molecule can be obtained.

### BRIEF DESCRIPTION OF THE DRAWINGS

The above-mentioned object, other objects, features, and advantages will become more apparent from preferred embodiments explained below by reference to the attached drawing.

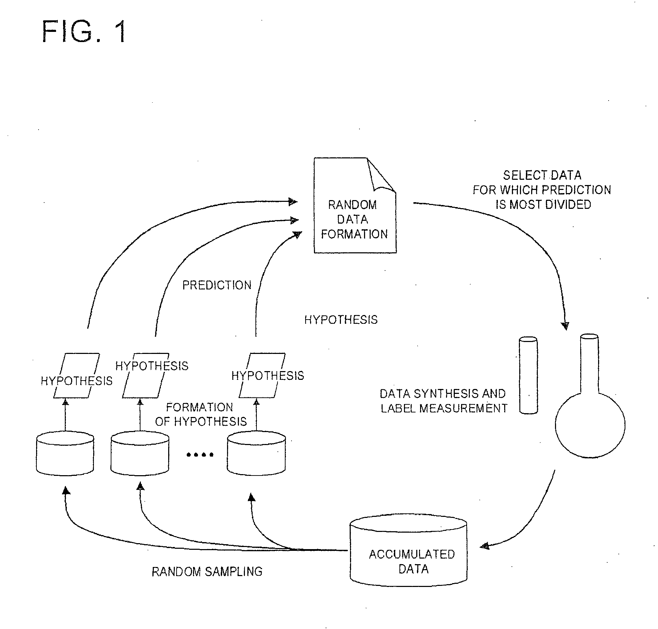

[FIG. 1] A schematic drawing for explaining an active learning experiment design used in an embodiment.

### BEST MODE FOR CARRYING OUT THE INVENTION

Modes for carrying out the present disclosure are explained below by reference to a drawing. In all the drawings, the same constitutional elements are denoted by the same reference numerals and symbols, so that the explanation will not be repeated.

### Embodiment 1

In this embodiment a peptide that contains an amino acid sequence for which the binding to an HLA molecule, predicted by a hypothesis obtained using an active learning experiment method (Japanese Patent Application Laid-open No. H11-316754 (1999)), is 3 or greater in terms of a -log Kd value, and consists of not less than 8 and not more than 11 amino acid residues is used as a candidate for an HLA-binding peptide. As a result of a binding experiment, it has been confirmed that these peptides are actually HLA-binding peptides.

As a result, a large number of HLA-binding peptides that have excellent properties in binding to an HLA-A type molecule because they contain an amino acid sequence for which the binding to the HLA molecule in terms of a - log Kd value is 3 or greater could be obtained efficiently.

Specifically, the HLA-binding peptide related to this embodiment is an HLA-binding peptide that binds to an HLA-A type molecule, contains at least one type of amino acid sequence selected from the group consisting of SEQ ID NOS: 1 to 60, which will be described later, and consists of not less than 8 and not more than 11 amino acid residues.

Among human HLA-A types, about 50% of Japanese people have the HLA-A24 type. Many European and American people, such as German people, have the HLA-A2 type.

All of these sequences herein mentioned are sequences consisting of 9 amino acid residues contained in a certain genome protein of cancer antigen WT1.

The sequences of SEQ ID NOS: 1 to 30 are given in Table 1 below.

**TABLE. 1**

| HLA-A24-BINDING PEPTIDE | | | | |
|---|---|---|---|---|
| SEQ ID No | AMINO ACID SEQUENCE | PREDICTED SCORE | SEQ NAME | BINDING EXPERIMENT DATA |
| 1 | DFKDCERRF | 5.6825 | 356 | 4.4035 |
| 2 | ALLPAVPSL | 5.5395 | 10 | 9.1624 |
| 3 | YRIHTHGVF | 5.4392 | 286 | 4.3926 |
| 4 | PPPPPPHSF | 5.3618 | 63 | 4.3968 |
| 5 | DVRDLNALL | 5.2751 | 4 | 4.4457 |
| 6 | SFIKQEPSW | 5.2543 | 70 | 8.1647 |
| 7 | PPPPPHSFI | 5.1407 | 64 | 4.3968 |
| 8 | FTVHFSGQF | 5.1158 | 92 | |
| 9 | SCLESQPAI | 5.0313 | 136 | |
| 10 | NMTKLQLAL | 5.0204 | 441 | 6.3753 |
| 11 | RTPYSSDNL | 5.0068 | 218 | |
| 12 | CLSAFTVHF | 4.9901 | 88 | |
| 13 | QCKTCQRKF | 4.93 | 384 | |
| 14 | ILOGAQYRI | 4.9165 | 280 | 5.9004 |
| 15 | YFKLSHLQM | 4.9072 | 334 | |
| 16 | TGKTSEKPF | 4.8891 | 406 | |
| 17 | TPSHHAAQF | 4.8697 | 163 | |
| 18 | NQGYSTVTF | 4.8546 | 146 | |
| 19 | AYPGCNKRY | 4.794 | 326 | |
| 20 | ASSGQARMF | 4.7598 | 120 | |
| 21 | DELVRHHNM | 4.7554 | 429 | |
| 22 | AGACRYGPF | 4.7495 | 104 | |
| 23 | AFTVHFSGQ | 4.7382 | 91 | |
| 24 | SCTGSQALL | 4.7364 | 208 | |
| 25 | AEPHEEQCL | 4.7332 | 81 | |
| 26 | LLLRTPYSS | 4.7304 | 215 | |
| 27 | AAQFPNHSF | 4.7135 | 168 | |
| 28 | HTHGVFRGI | 4.6454 | 289 | |
| 29 | NLYQMTSQL | 4.5632 | 225 | 4.3769 |
| 30 | QYRIHTHGV | 4.5036 | 285 | 5.6157 |

The sequences of SEQ ID NOS: 1 to 30 are sequences consisting of 9 amino acid residues contained in a certain genome protein of the cancer antigen WT1. The sequences of SEQ ID NOS: 1 to 30 are sequences predicted by the above-mentioned method to have superior binding to an HLA-A24 molecule. SEQ ID NOS: 1 to 30 are arranged in decreasing binding order. That is, SEQ ID NO: 1 is the sequence that is predicted to have the best binding. A predicted score for binding to the HLA-A24 molecule and binding experiment data for each sequence are expressed in the form of -log Kd values.

The sequences of SEQ ID NOS: 31 to 60 are given in Table 2 below.

**TABLE. 2**

| HLA-A2-BINDING PEPTIDE | | | | |
|---|---|---|---|---|
| SEQ ID No | AMINO ACID SEQUENCE | PREDICTED SCORE | SEQ NAME | BINDING EXPERIMENT DATA |
| 31 | NLGATLKGV | 5.4061 | 242 | 5.088 |
| 32 | DLNALLPAV | 5.3406 | 7 | |
| 33 | ALLPAVPSL | 5.299 | 10 | 7.8445 |
| 34 | SCLESQPAI | 5.2547 | 136 | |
| 35 | LLPAVPSLG | 5.0553 | 11 | |
| 36 | VLDFPAPPGA | 5.0241 | 37 | |
| 37 | SGAAQWAPV | 5.0084 | 29 | |
| 38 | ALLLRTPYS | 4.9954 | 214 | 5.3284 |
| 39 | QQYSVPPPV | 4.8665 | 191 | 5.4895 |
| 40 | ILCGAQYRI | 4.8389 | 280 | 5.1242 |
| 41 | LLLRTPYSS | 4.797 | 215 | |
| 42 | FGPPPPSQA | 4.7892 | 112 | |
| 43 | VAAGSSSSV | 4.773 | 250 | 5.0021 |
| 44 | NLYQMTSQL | 4.7114 | 225 | 5.8268 |
| 45 | VPGVAPTLV | 4.669 | 303 | |
| 46 | EQCLSAFTV | 4.6626 | 86 | |
| 47 | QYRIHTHGV | 4.6069 | 285 | |
| 48 | ALPVSGAAQ | 4.5992 | 25 | |
| 49 | NMTKLQLAL | 4.5771 | 441 | 5.4308 |
| 50 | IRNQGYSTV | 4.5211 | 144 | |
| 51 | GVFRGIQDV | 4.4566 | 292 | 5.8494 |
| 52 | NAPYLPSCL | 4.4186 | 130 | |
| 53 | FMCAYPGCN | 4.4085 | 323 | |
| 54 | GQFTGTAGA | 4.4011 | 98 | |
| 55 | SQLECMTWN | 4.3733 | 231 | |
| 56 | CLESQPAIR | 4.3672 | 137 | |
| 57 | TLKGVAAGS | 4.3527 | 246 | |
| 58 | KKFARSDEL | 4.3526 | 423 | |
| 59 | SLGQPAPPP | 4.3384 | 50 | |
| 60 | TLVRSASET | 4.271 | 309 | |

The sequences of SEQ ID NOS: 31 to 60 are sequences consisting of 9 amino acid residues contained in a certain genome protein of the cancer antigen WT1. The sequences of SEQ ID NOS: 31 to 60 are sequences predicted by the above-mentioned method to have superior binding to an HLA-A2 molecule. SEQ ID NOS: 31 to 60 are arranged in decreasing binding order. That is, SEQ ID NO: 31 is the sequence that is predicted to have the best binding. A predicted score for binding to the HLA-A2 molecule and binding experiment data for each sequence are expressed in the form of -log Kd values.

Although details are described later, it is clear that there is a correlation between the predicted score and the binding experiment data. That is, although there are slight errors, it can be said that a peptide that is predicted by the above-mentioned method to have high binding to the HLA-A molecule is found experimentally to have high binding to the HLA-A molecule.

Since there is no conventional technique for discovering an HLA-binding peptide by utilizing such an experimental design method, there are only a very small number of HLA-binding peptides that have been experimentally confirmed to have HLA-binding properties. Because of this, even when a peptide consisting of 9 amino acid residues is randomly synthesized by a conventional method and subjected to an experiment to find out if it binds to an HLA molecule, there is a probability of only about 1 in 100 of finding one that has a binding, in terms of a -log Kd value, exceeding 6.

In accordance with this embodiment, since the technique of finding an HLA-binding peptide by utilizing the experimental design method is used, as described above, as many as 60 sequences of HLA-binding peptides can be found. Furthermore, when the binding of some of the HLA-binding peptides obtained is experimentally examined, it is confirmed that all of the sequences that have been subjected to the experiment exhibit an excellent binding to HLA that is equal to or higher than that predicted.

Among these sequences, an HLA-binding peptide containing at least one type of amino acid sequence selected from the group consisting of SEQ ID NOS: 1, 2, 3, 4, 5, 6, 7, 10, 14, 29, 30, 31, 33, 38, 39, 40, 43, 44, 49, and 51 is experimentally confirmed to bind to a human HLA-A type molecule. It can therefore be said with certainty that it is an HLA-binding peptide that has excellent properties in binding to a human HLA-A type molecule.

The binding to an HLA molecule of the HLA-binding peptide related to the present embodiment is 3 or greater in terms of a -log Kd value, particularly preferably 5 or greater, and more preferably 5.4 or greater.

In the field of biochemistry, it is known that a binding ability, in terms of a -log Kd value, of about 3 is the threshold level for whether or not a peptide actually binds to an MHC. Therefore, if the binding to an HLA molecule, in terms of a -log Kd value, is 3 or greater, it can be said that it is an HLA-binding peptide.

Furthermore, if the binding to an HLA molecule, in terms of a -log Kd value, is 5 or greater, since the peptide obtained has excellent properties in binding to the HLA molecule, it can suitably be used for development of an effective therapeutic drug, preventive drug, and the like for an immune disease and the like.

Moreover, if the binding to an HLA molecule, in terms of a -log Kd value, is 5.4 or greater, the peptide obtained has particularly good properties in binding to the HLA molecule, and it can suitably be used for the development of an even more effective therapeutic drug, prophylactic drug, and the like for an immune disease and the like.

Furthermore, it may be arranged that the HLA-binding peptide related to the present embodiment consists of not less than 8 and not more than 11 amino acid residues.

In this way, if the peptide consists of not less than 8 and not more than 11 amino acid residues, it has excellent properties in binding to an HLA molecule. Furthermore, the cytotoxic T lymphocyte (CTL) specifically recognizes a cancer antigen specific to a cancer cell (CTL epitope) consisting of 8 to 11 amino acids presented in an HLA class I molecule on the surface of a cancer cell, and eliminates the cancer cell by damaging only the cancer cell. It is important to prepare such a CTL epitope consisting of 8 to 11 amino acids that is specific to a cancer cell and the like in order to prepare a vaccine for therapy or prevention against the cancer and the like.

For example, the above-mentioned HLA-binding peptide may be a peptide consisting of amino acid residues alone, but it is not particularly limited thereto. For example, it may be an HLA-binding peptide precursor that is optionally modified with a sugar chain or a fatty acid group and the like as long as the effects of the present invention are not impaired. Such a precursor is subjected to a change involving digestion by a digestive enzyme and the like in a living mammalian body such as in a human digestive organ to become an HLA-binding peptide, thus exhibiting similar effects to those shown by the above-mentioned HLA-binding peptide.

Furthermore, the above-mentioned HLA-binding peptide may be a peptide that binds to a human HLA-A24 molecule.

In accordance with this constitution, since a peptide is obtained that binds to an HLA-A24 molecule, which is often seen in Asian people, such as Japanese people, it can be utilized in the development of a therapeutic drug, a preventive drug, and the like that is particularly effective for Asian people, such as Japanese people.

Furthermore, the above-mentioned HLA-binding peptide may be a peptide that binds to a human HLA-A2 molecule.

In accordance with this constitution, since a peptide is obtained that binds to an HLA-A2 molecule, which is often seen in European and American people, it can be utilized in the development of a therapeutic drug, a preventive drug, and the like that is particularly effective for European and American people.

The amino acid sequence contained in the above-mentioned HLA-binding peptide may be an amino acid sequence derived from cancer antigen WT1 protein, but it is not particularly limited thereto. For example, it may be an amino acid sequence derived from an HIV protein, or an amino acid sequence derived from a cedar pollen protein and the like. Moreover, it may contain an amino acid sequence derived from a protein having other pathogenicity or allergenicity.

### Embodiment 2

In accordance with this embodiment, there is provided an HLA-binding peptide that binds to an HLA-A type molecule, contains an amino acid sequence formed by deletion, substitution, or addition of one or two amino acid residues of the amino acid sequence contained in the above-mentioned HLA-binding peptide, and consists of not less than 8 and not more than 11 amino acid residues.

As described later, even though the constitution includes an amino acid sequence formed by deletion, substitution, or addition of one or a few amino acid residues of a specific amino acid sequence that binds to an HLA-A type molecule, similar effects to those of the HLA-binding peptide related to the above-mentioned embodiment 1 are exhibited.

That is, it can be predicted that even an amino acid sequence formed by deletion, substitution, or addition of one or two amino acid residues of an amino acid sequence shown in SEQ ID NOS: 1 to 50 that has excellent properties in binding to an HLA-A24 molecule will show excellent HLA-binding properties in a similar manner.

From another viewpoint, it can be predicted that even an amino acid sequence formed by deletion, substitution, or addition of one or a few amino acid residues of an amino acid sequence predicted by the above-mentioned method to have excellent properties in binding to an HLA-A24 molecule will show excellent HLA-binding properties in a similar manner. The amino acid residues that are substituted are preferably amino acid residues having similar properties to each other, such as both being hydrophobic amino acid residues.

Moreover, the HLA-binding peptides described in Embodiment 1 and Embodiment 2 can be produced using a method known to a person skilled in the art. For example, they may be artificially synthesized by a solid-phase method or a liquid-phase method. Alternatively, these HLA-binding peptides may be produced by expressing them from a DNA segment or a recombinant vector coding for these HLA-binding peptides. These HLA-binding peptides thus obtained can be identified by a method known to a person skilled in the art. For example, identification is possible by use of Edman degradation, mass spectrometry, and the like.

### Embodiment 3

In accordance with the present embodiment, there is provided a DNA segment containing a DNA sequence coding for the above-mentioned HLA-binding peptide. Since the DNA segment related to the present embodiment contains a specific DNA sequence, it can express the above-mentioned HLA-binding peptide.

When the above-mentioned HLA-binding peptide is expressed by using the DNA segment related to the present embodiment, expression may be carried out by incorporating this DNA segment into a cell, or expression may be carried out by using a commercial artificial protein expression kit.

Furthermore, continuous expression may be carried out by incorporating the above-mentioned DNA segment into, for example, a human cell. Because of this, an HLA-binding peptide can be made to be present continuously within a cell by incorporating a DNA segment coding for the HLA-binding peptide into the cell rather than incorporating the HLA-binding peptide itself into the cell. When an HLA-binding peptide is used as a vaccine, such an ability to express continuously is advantageous in terms of enhancing the efficacy of the vaccine.

Moreover, the DNA segment related to the present embodiment can be produced by a method known to a person skilled in the art. For example, it may be artificially synthesized by means of a commercial DNA synthesizer and the like. Alternatively, it may be segmented from the HCV genome by using a restriction enzyme and the like. Alternatively, it may be amplified from the HCV genome by a PCR method using a primer. The DNA segment thus obtained may be identified using a method known to a person skilled in the art. For example, it may be identified by a commercial DNA sequencer.

### Embodiment 4

In accordance with the present embodiment, there is provided a recombinant vector that contains a DNA sequence coding for the above-mentioned HLA-binding peptide. Since the recombinant vector related to the present embodiment contains a specific DNA sequence, the above-mentioned HLA-binding peptide can be expressed.

When the above-mentioned HLA-binding peptide is expressed by using the recombinant vector related to the present embodiment, expression may be carried out by incorporating this recombinant vector into a cell, or expression may be carried out by using a commercial artificial protein expression kit.

Furthermore, continuous expression may be carried out by incorporating the above-mentioned recombinant vector into, for example, a human cell. Because of this, the HLA-binding peptide can be made to be present continuously within a cell by incorporating a recombinant vector coding for the HLA-binding peptide into the cell rather than incorporating the HLA-binding peptide itself into the cell. When the HLA-binding peptide is used as a vaccine, such an ability to express continuously is advantageous in terms of enhancing the efficacy of the vaccine.

Furthermore, in the above-mentioned recombinant vector, the amount of HLA-binding peptide expressed can be controlled with high precision by the use of a certain sequence in a regulatory region involved in transcription and expression, such as a promoter region upstream of a DNA sequence coding for the above-mentioned HLA-binding peptide. Moreover, the number of copies of a recombinant vector in a cell can be controlled with high precision by the use of a certain sequence in a regulatory region involved in replication, such as the origin region of the recombinant vector.

Furthermore, the above-mentioned recombinant vector may freely contain a sequence other than the DNA sequence coding for the above-mentioned HLA-binding peptide. For example, it may contain a sequence of a marker gene such as a drug resistance gene.

Moreover, the recombinant vector related to the present embodiment can be produced using a method known to a person skilled in the art. For example, it may be obtained by cleaving a multicloning site of a commercial vector such as pBR322 or pUC19 at a certain restriction enzyme site, and inserting the above-mentioned DNA segment into the site and carrying out ligation. Furthermore, the recombinant vector thus obtained can be identified using a method known to a person skilled in the art. For example, it can be confirmed by agarose gel electrophoresis whether or not the length of the DNA segment cleaved by a predetermined restriction enzyme coincides with the restriction map of a commercial vector such as pBR322 or pUC19 and, furthermore, it can be identified by a DNA sequencer and the like whether or not the above-mentioned DNA sequence is contained in the DNA sequence cut out from the multicloning site.

The constitutions of the present disclosure are explained above, but any combination of these constitutions is also effective as an embodiment of the present disclosure. Furthermore, conversion of the expression of the present disclosure into another category is also effective as an embodiment of the present disclosure.

For example, in the above-mentioned embodiments, the HLA-binding peptide contains an amino acid sequence derived from a certain genome protein (SEQ ID NO: 61) of cancer antigen WT1, but an HLA-binding peptide for a pathogen other than WT1, such as an HIV virus, may be employed, and an HLA-binding peptide containing an amino acid sequence derived from a protein, such as a cedar pollen allergen and the like, may be employed.

In this way, if an amino sequence that is predicted by the above-mentioned method to have excellent HLA-binding properties is contained, it can be expected that it will exhibit excellent HLA-binding properties in a similar way when confirmation is carried out experimentally. Because of this, these HLA-binding peptides can suitably be used mainly for the therapy or prevention of infectious diseases (influenza, SARS, HIV, HCV and the like), and also for cancer immunotherapy, allergic diseases (pollen allergy (hay fever), rheumatism, atopy, asthma and the like), autoimmune diseases, and the like.

### (Example 1)

The invention is further explained below by reference to Examples.

Specifically, procedures of prediction, experiment, and evaluation in the present examples were carried out based on an active learning experiment design, and in general the following steps were repeated. A schematic drawing for the active learning experiment design employed here is shown in FIG. 1.
(1) A trial of a lower-order learning algorithm, which will be described later, was carried out once. That is, a plurality of hypotheses were generated by random sampling from accumulated data and, with regard to randomly expressed candidate query points (peptides), a point that showed the largest distribution of predicted values was selected as a query point to be subjected to an experiment.
(2) The peptide at the selected query point was prepared by a synthesis and purification method, which will be described later, and the actual binding ability was measured by an experiment, which will be described later, and added to accumulated data.

In the present example, as the lower-order learning algorithm, a supervised learning algorithm of a Hidden Markov Model was used, and 20 to 30 types of peptides were predicted and selected per experiment by starting with the initial data for 223 types of peptides; the above-mentioned procedure was repeated four times, and a total of 341 data points were obtained.

More specifically, in the active learning method of the present example, 20 to 30 types of peptides containing an amino acid sequence in which 9 of 20 types of amino acids were arranged were designed and synthesized per experiment. The strength of binding (binding ability) thereof to an HLA molecule was measured. The binding ability (Kd value in molar concentration) was obtained as an experimental result. When the binding ability was high, the peptide was selected as a candidate for an HLA-binding peptide that could be used as a material for a vaccine.

The results thus obtained were inputted into a learning system equipped with a learning machine employing the Hidden Markov Model as a mathematical algorithm, and rules were created. The learning machine sampled different results to prepare the rules. The rules expressed by the learning machine had different constitutions. The rules thus obtained and experimental data were stored as needed as accumulated data.

From among more than 500 billion (= 20⁹) peptide sequences, candidates for a subsequent experiment were selected by the rules, and the above-mentioned process was repeated. In this stage, different rules were applied to experimental candidates, and the candidates for which predictions of the experimental results were divided were subjected to experiment. In this way, since the candidates for which predictions of the experimental results were divided were subjected to subsequent experiment, the final precision of the prediction was increased.

In this way, a plurality of learning machines carried out selective sampling in which samples that would give different predictions were selected as experimental candidates, information could be gained efficiently, and a hypothesis (rule) with high precision could be obtained. Repeating the above-mentioned process four times gave excellent results as in Examples described later. Repeating it seven times or more gave even better results.

In accordance with such an active learning method, the number of repetitions of the binding experiment for peptides consisting of 9 amino acid residues, which would otherwise have to be carried out for the 500 billion or more combinations of all the candidates for HLA-binding peptides, could be reduced. In the active learning method, a rule was formed by experiment, and the experiment was repeated for tens of sequence candidates that were predicted by applying the rule. Because of this, the number of experiments could be cut, and the time and cost of the initial screening could be greatly reduced.

Furthermore, the hit rate for prediction of the binding of a peptide to HLA by the rule obtained by the active learning method reached 70 to 80%, whereas the hit rate by other known techniques such as the anchor method was as low as about 30%.

### <Synthesis and purification of peptide>

A peptide was manually synthesized by the Merrifield solid-phase method using Fmoc amino acids. After deprotection, reverse phase HPLC purification was carried out using a C18 column to give a purity of 95% or higher. Identification of the peptide and confirmation of its purity were carried out using a MALDI-TOF mass spectrometer (Voyager DE RP, PerSeptive). Quantitative analysis of the peptide was carried out by a Micro BCA assay (Pierce Corp.) using BSA as a standard protein.

### <Experiment of binding peptide to HLA-A24 molecule>

The ability of a peptide to bind to an HLA-A24 molecule, which is a product of the HLA-A*2402 gene, was measured using C1R-A24 cells expressing the HLA-A24 molecule (cells prepared by Professor Masafumi Takiguchi, Kumamoto University being supplied with permission by Assistant Professor Masaki Yasukawa, Ehime University).

C1R-A24 cells were first exposed to acidic conditions at a pH of 3.3 for 30 seconds, thus dissociating and removing a light chain β2m, which is associated with HLA class I molecules in common, and an endogenous peptide originally bound to the HLA-A*2402 molecule. After neutralization, purified β2m was added to C1R-A24 cells, the obtained product was added to serial dilutions of a peptide, and incubated on ice for 4 hours. Staining was carried out using fluorescently labeled monoclonal antibody 17A12, which recognizes association (MHC-pep) of the three members, that is, HLA-A*2402 molecule, the peptide, and β2m, which had reassociated during the incubation.

Subsequently, the MHC-pep count per C1R-A24 cell (proportional to the strength of fluorescence of the above-mentioned fluorescent antibody) was quantitatively measured using an FACScan fluorescence-activated cell sorter (Becton Dickinson Biosciences). A binding dissociation constant Kd value between the HLA-A24 molecule and the peptide was calculated from the average strength of fluorescence per cell by a published method (Udaka et al., Immunogenetics, 51, 816-828, 2000).

### <Experiment of binding peptide to HLA-A2 molecule>

The ability of a peptide to bind to an HLA-A2 molecule, which is a product of the HLA-A*0201 gene, was measured using strain JY cells expressing the HLA-A*0201.

JY cells were first exposed to acidic conditions at a pH of 3.8 for 30 seconds, thus dissociating and removing a light chain β2m and an endogenous peptide, which were noncovalently associated with the HLA-A*0201 molecule. After neutralization, a reassociation experiment was carried out.

The above-mentioned JY cells and the purified β2m were added to stepped dilutions of peptide for which the binding ability would be measured, and incubation was carried out on ice for 4 hours. HLA-A*0201 molecules that had reassociated up to this point were stained using the associating type specific fluorescently-labeled monoclonal antibody BB7.2.

Subsequently, the amount of fluorescence per cell was measured using a flow cytometer and a dissociation constant Kd value in molar concentration was calculated by a published method (Udaka et al., Immunogenetics, 51, 816-828, 2000).

### <Evaluation results>

The prediction results and the experimental results shown in Tables 1 and 2 above were obtained.

The sequences of SEQ ID NOS: 1 to 30 in Table 1 are sequences consisting of 9 amino acid residues contained in the full-length sequence of a certain protein of WT1 registered in the GENBANK. Furthermore, the sequences of SEQ ID NOS: 1 to 30 are sequences having superior binding to an HLA-A24 molecule as predicted by a hypothesis obtained by the experimental design method explained in Embodiment 1. SEQ ID NOS: 1 to 30 are arranged in decreasing binding order. That is, SEQ ID NO: 1 is the sequence that is predicted to have the best binding. The full-length amino acid sequence of the certain genome protein of WT1 is shown in SEQ ID NO: 61.

Table 1 shows the 30 amino acid sequences that had superior scores in the predicted results obtained using the above-mentioned prediction program, the predicted score, and the corresponding binding experiment data. All of the binding experiment data were obtained by artificially synthesizing peptide sequences of WT1 by the above-mentioned synthetic method.

Furthermore, the sequences of SEQ ID NOS: 31 to 60 in Table 2 are sequences consisting of 9 amino acid residues contained in the full-length sequence of a certain protein of WT1 registered in the GenBank. Furthermore, the sequences of SEQ ID NOS: 31 to 60 are sequences having superior binding to an HLA-A2 molecule as predicted by a hypothesis obtained by the experimental design method explained in Embodiment 1. SEQ ID NOS: 31 to 60 are arranged in decreasing binding order. That is, SEQ ID NO: 31 is the sequence that is predicted to have the best binding.

The full-length sequence of the certain protein of WT1 is shown in SEQ ID NO: 61(MGSDVRDLNALLPAVPSLGGGGGCALP VSGAAQWAPVLDFAPPGASAYGSLGGPAPPPAPPPPPPPPPHSFIKQEPSWGGAEPH EEQCLSAFTVHFSGQFTGTAGACRYGPFGPPPPSQASSGQARMFPNAPYLPSCLESQ PAIRNQGYSTVTFDGTPSYGHTPSHHAAQFPNHSFKHEDPMGQQGSLGEQQYSVPPP VYGCHTPTDSCTGSQALLLRTPYSSDNLYQMTSQLECMTWNQMNLGATLKGVAAGSS SSVKWTEGQSNHSTGYESDNHTTPILCGAQYRIHTHGVFRGIQDVRRVPGVAPTLVR SASETSEKRPFMCAYPGCNKRYFKLSHLQMHSRKHTGEKPYQCDFKDCERRFSRSDQ LKRHQRRHTGVKPFQCKTCQRKFSRSDHLKTHTRTHTGKTSEKPFSCRWPSCQKKFA RSDELVRHHNMHQRNMTKLQLAL).

Table 2 shows the 30 amino acid sequences that had superior scores in the predicted results obtained using the above-mentioned prediction program, the predicted score, and the corresponding binding experiment data. All of the binding experiment data were obtained by artificially synthesizing peptide sequences of WT1 by the above-mentioned synthetic method.

It can be predicted that any of the peptide sequences formed by substitution of one or two amino acid residues with each other will show excellent binding to an HLA-A molecule. In conclusion, even an amino acid sequence formed by deletion, substitution, or addition of one or a few amino acid residues of an amino acid sequence that has excellent properties in binding to an HLA-A molecule shown by SEQ ID NOS: 1 to 60 can be predicted to similarly show excellent HLA-binding properties.

From another viewpoint, even an amino acid sequence formed by deletion, substitution, or addition of one or a few amino acid residues of an amino acid sequence that has excellent properties in binding to an HLA-A molecule as predicted by the hypothesis obtained by the experimental design method explained in Embodiment 1 similarly can be said to show excellent HLA-binding properties. The amino acid residues that are substituted are preferably amino acid residues that have similar properties to each other, such as the two being hydrophobic amino acid residues.

Udaka, who is one of the inventors, et al have already reported that even a peptide sequence formed by substitution of one or two amino acid residues in the peptide sequence will similarly show excellent binding properties to an antigen-presenting molecule.
1. "Decrypting the structure of MHC-I restricted CTL epitopes with complex peptide libraries." Keiko Udaka, Karl-Heinz Wiesmuller, Stefan Kienle, Gunter Jung and Peter Walden. J. Exp. Med. 181, 2097-2108 (1995).
2. "Tolerance to amino acid variations in peptides binding to the MHC class I protein H-2Kb." Keiko Udaka, Karl-Heinz Wiesmuller, Stefan Kienle, Gunter Jung and Peter Walden. J. Biol. Chem. 270, 24130-24134 (1995).
3. "Self MHC-restricted peptides recognized by all alloreactive T lymphocyte clone." Keiko Udaka, Karl-Heinz Wiesmuller, Stefan Kienle, Gunter Jung and Peter Walden. J. Immunol. 157, 670-678 (1996).

Therefore, it can be predicted that even the cancer antigen WT1-derived peptide described in the present invention or even the above-mentioned peptide sequence formed by substitution of one or two amino acid residues in the peptide sequence will similarly show excellent binding properties to the HLA-A molecule.

The invention is explained above by reference to Examples.

**Furthermore, the present disclosure relates to the following items:**
1. An HLA-binding peptide binding to an HLA-A type molecule, said HLA-binding peptide comprising:
   at least one type of amino acid sequence selected from the group consisting of SEQ ID NOS: 1 to 60, and
   not less than 8 and not more than 11 amino acid residues.
2. The HLA-binding peptide as set forth in **Item** 1, comprising at least one type of amino acid sequence selected from the group consisting of SEQ ID NOS: 1, 2, 3, 4, 5, 6, 7, 10, 14, 29, 30, 31, 33, 38, 39, 40, 43, 44, 49, and 51.
3. An HLA-binding peptide binding to an HLA-A type molecule, said HLA-binding peptide comprising:
   an amino acid sequence formed by deletion, substitution, or addition of one or two amino acid residues of said amino acid sequence contained in the HLA-binding peptide as set forth in **Item** 1 or 2, and
   not less than 8 and not more than 11 amino acid residues.
4. The HLA-binding peptide as set forth in any one of **Items** 1 to 3, wherein said HLA-binding peptide binds to a human HLA-A24 molecule.
5. The HLA-binding peptide as set forth in any one of Items 1 to 3, wherein said HLA-binding peptide binds to a human HLA-A2 molecule.
6. A DNA segment comprising a DNA sequence coding for the HLA-binding peptide as set forth in any one of **Items** 1 to 5.
7. A recombinant vector comprising a DNA sequence coding for the HLA-binding peptide as set forth in any one of **Items** 1 to 5.
8. An HLA-binding peptide precursor changing within a mammalian body into the HLA-binding peptide as set forth in any one of **Items** 1 to 5.

## Claims

1. An HLA-binding peptide consisting of SEQ ID NO: 38 which binds to a human HLA-A2 molecule.

## Patentansprüche

1. HLA-bindendes Peptid bestehend aus SEQ ID NO: 38, das an ein humanes HLA-A2-Molekül bindet.

## Revendications

1. Peptide de liaison à HLA consistant en SEQ ID NO: 38 qui se lie à une molécule HLA-A2 humaine.


### SEQUENCE LISTING

<110> NEC Corporation
   Kochi University
<120> HLA binding peptide, and DNA fragment and recombinat vector coding
<130> N1435 EP/1 S3
<140>
   <141> 2005-09-13
<150> JP 2004-272385
   <151> 2005-02-23
<160> 61
<170> PatentIn version 3.1
<210> 1
   <211> 9
   <212> PRT
   <213> Homo sapiens
<400> 1
   Asp Phe Lys Asp Cys Glu Arg Arg Phe
   1               5
<210> 2
   <211> 9
   <212> PRT
   <213> Homo sapiens
<400> 2
   Ala Leu Leu Pro Ala Val Pro Ser Leu
   1               5
<210> 3
   <211> 9
   <212> PRT
   <213> Homo sapiens
<400> 3
   Tyr Arg Ile His Thr His Gly Val Phe
   1 5
<210> 4
   <211> 9
   <212> PRT
   <213> Homo sapiens
<400> 4
   Pro Pro Pro Pro Pro Pro His Ser Phe
   1 5
<210> 5
   <211> 9
   <212> PRT
   <213> Homo sapiens
<400> 5
   Asp Val Arg Asp Leu Asn Ala Leu Leu
   1 5
<210> 6
   <211> 9
   <212> PRT
   <213> Homo sapiens
<400> 6
   Ser Phe Ile Lys Gln Glu Pro Ser Trp
   1 5
<210> 7
   <211> 9
   <212> PRT
   <213> Homo sapiens
<400> 7
   Pro Pro Pro Pro Pro His Ser Phe Ile
   1 5
<210> 8
   <211> 9
   <212> PRT
   <213> Homo sapiens
<400> 8
   Phe Thr Val His Phe Ser Gly Gln Phe
   1 5
<210> 9
   <211> 9
   <212> PRT
   <213> Homo sapiens
<400> 9
   Ser Cys Leu Glu Ser Gln Pro Ala Ile
   1 5
<210> 10
   <211> 9
   <212> PRT
   <213> Homo sapiens
<400> 10
   Asn Met Thr Lys Leu Gln Leu Ala Leu
   1 5
<210> 11
   <211> 9
   <212> PRT
   <213> Homo sapiens
<400> 11
   Arg Thr Pro Tyr Ser Ser Asp Asn Leu
   1 5
<210> 12
   <211> 9
   <212> PRT
   <213> Homo sapiens
<400> 12
   Cys Leu Ser Ala Phe Thr Val His Phe
   1 5
<210> 13
   <211> 9
   <212> PRT
   <213> Homo sapiens
<400> 13
   Gln Cys Lys Thr Cys Gln Arg Lys Phe
   1 5
<210> 14
   <211> 9
   <212> PRT
   <213> Homo sapiens
<400> 14
   Ile Leu Cys Gly Ala Gln Tyr Arg Ile
   1 5
<210> 15
   <211> 9
   <212> PRT
   <213> Homo sapiens
<400> 15
   Tyr Phe Lys Leu Ser His Leu Gln Met
   1 5
<210> 16
   <211> 9
   <212> PRT
   <213> Homo sapiens
<400> 16
   Thr Gly Lys Thr Ser Glu Lys Pro Phe
   1 5
<210> 17
   <211> 9
   <212> PRT
   <213> Homo sapiens
<400> 17
   Thr Pro Ser His His Ala Ala Gln Phe
   1 5
<210> 18
   <211> 9
   <212> PRT
   <213> Homo sapiens
<400> 18
   Asn Gln Gly Tyr Ser Thr Val Thr Phe
   1 5
<210> 19
   <211> 9
   <212> PRT
   <213> Homo sapiens
<400> 19
   Ala Tyr Pro Gly Cys Asn Lys Arg Tyr
   1 5
<210> 20
   <211> 9
   <212> PRT
   <213> Homo sapiens
<400> 20
   Ala Ser Ser Gly Gln Ala Arg Met Phe
   1 5
<210> 21
   <211> 9
   <212> PRT
   <213> Homo sapiens
<400> 21
   Asp Glu Leu Val Arg His His Asn Met
   1 5
<210> 22
   <211> 9
   <212> PRT
   <213> Homo sapiens
<400> 22
   Ala Gly Ala Cys Arg Tyr Gly Pro Phe
   1 5
<210> 23
   <211> 9
   <212> PRT
   <213> Homo sapiens
<400> 23
   Ala Phe Thr Val His Phe Ser Gly Gln
   1 5
<210> 24
   <211> 9
   <212> PRT
   <213> Homo sapiens
<400> 24
   Ser Cys Thr Gly Ser Gln Ala Leu Leu
   1               5
<210> 25
   <211> 9
   <212> PRT
   <213> Homo sapiens
<400> 25
   Ala Glu Pro His Glu Glu Gln Cys Leu
   1               5
<210> 26
   <211> 9
   <212> PRT
   <213> Homo sapiens
<400> 26
   Leu Leu Leu Arg Thr Pro Tyr Ser Ser
   1               5
<210> 27
   <211> 9
   <212> PRT
   <213> Homo sapiens
<400> 27
   Ala Ala Gln Phe Pro Asn His Ser Phe
   1               5
<210> 22
   <211> 9
   <212> PRT
   <213> Homo sapiens
<400> 28
<210> 29
   <211> 9
   <212> PRT
   <213> Homo sapiens
<400> 29
   Asn Leu Tyr Gln Met Thr Ser Gln Leu
   1               5
<210> 30
   <211> 9
   <212> PRT
   <213> Homo sapiens
<400> 30
   Gln Tyr Arg Ile His Thr His Gly Val
   1               5
<210> 31
   <211> 9
   <212> PRT
   <213> Homo sapiens
<400> 31
   Asn Leu Gly Ala Thr Leu Lys Gly Val
   1               5
<210> 32
   <211> 9
   <212> PRT
   <213> Homo sapiens
<400> 32
   Asp Leu Asn Ala Leu Leu Pro Ala Val
   1               5
<210> 33
   <211> 9
   <212> PRT
   <213> Homo sapiens
<400> 33
   Ala Leu Leu Pro Ala Val Pro Ser Leu
   1               5
<210> 34
   <211> 9
   <212> PRT
   <213> Homo sapiens
<400> 34
   Ser Cys Leu Glu Ser Gln Pro Ala Ile
   1 5
<210> 35
   <211> 9
   <212> PRT
   <213> Homo sapiens
<400> 35
   Leu Leu Pro Ala Val Pro Ser Leu Gly
   1 5
<210> 36
   <211> 9
   <212> PRT
   <213> Homo sapiens
<400> 36
   Val Leu Asp Phe Ala Pro Pro Gly Ala
   1 5
<210> 37
   <211> 9
   <212> PRT
   <213> Homo sapiens
<400> 37
   Ser Gly Ala Ala Gln Trp Ala Pro Val
   1 5
<210> 38
   <211> 9
   <212> PRT
   <213> Homo sapiens
<400> 38
   Ala Leu Leu Leu Arg Thr Pro Tyr Ser
   1 5
<210> 39
   <211> 9
   <212> PRT
   <213> Homo sapiens
<400> 39
   Gln Gln Tyr Ser Val Pro Pro Pro Val
   1 5
<210> 34
   <211> 9
   <212> PRT
   <213> Homo sapiens
<400> 40
<210> 41
   <211> 9
   <212> PRT
   <213> Homo sapiens
<400> 41
   Leu Leu Leu Arg Thr Pro Tyr Ser Ser
   1 5
<210> 42
   <211> 9
   <212> PRT
   <213> Homo sapiens
<400> 42
   Phe Gly Pro Pro Pro Pro Ser Gln Ala
   1 5
<210> 43
   <211> 9
   <212> PRT
   <213> Homo sapiens
<400> 43
   Val Ala Ala Gly Ser Ser Ser Ser Val
   1 5
<210> 44
   <211> 9
   <212> PRT
   <213> Homo sapiens
<400> 44
   Asn Leu Tyr Gln Met Thr Ser Gln Leu
   1 5
<210> 45
   <211> 9
   <212> PRT
   <213> Homo sapiens
<400> 45
   Val Pro Gly Val Ala Pro Thr Leu Val
   1 5
<210> 46
   <211> 9
   <212> PRT
   <213> Homo sapiens
<400> 46
   Glu Gln Cys Leu Ser Ala Phe Thr Val
   1 5
<210> 47
   <211> 9
   <212> PRT
   <213> Homo sapiens
<400> 47
   Gln Tyr Arg Ile His Thr His Gly Val
   1 5
<210> 48
   <211> 9
   <212> PRT
   <213> Homo sapiens
<400> 48
   Ala Leu Pro Val Ser Gly Ala Ala Gln
   1 5
<210> 49
   <211> 9
   <212> PRT
   <213> Homo sapiens
<400> 49
   Asn Met Thr Lys Leu Gln Leu Ala Leu
   1 5
<210> 50
   <211> 9
   <212> PRT
   <213> Homo sapiens
<400> 50
   Ile Arg Asn Gln Gly Tyr Ser Thr Val
   1 5
<210> 51
   <211> 9
   <212> PRT
   <213> Homo sapiens
<400> 51
   Gly Val Phe Arg Gly Ile Gln Asp Val
   1 5
<210> 52
   <211> 9
   <212> PRT
   <213> Homo sapiens
<400> 52
   Asn Ala Pro Tyr Leu Pro Ser Cys Leu
   1 5
<210> 53
   <211> 9
   <212> PRT
   <213> Homo sapiens
<400> 53
   Phe Met Cys Ala Tyr Pro Gly Cys Asn
   1 5
<210> 54
   <211> 9
   <212> PRT
   <213> Homo sapiens
<400> 54
   Gly Gln Phe Thr Gly Thr Ala Gly Ala
   1 5
<210> 55
   <211> 9
   <212> PRT
   <213> Homo sapiens
<400> 55
   Ser Gln Leu Glu Cys Met Thr Trp Asn
   1 5
<210> 55
   <211> 9
   <212> PRT
   <213> Homo sapiens
<400> 56
<210> 57
   <211> 9
   <212> PRT
   <213> Homo sapiens
<400> 57
   Thr Leu Lys Gly Val Ala Ala Gly Ser
   1 5
<210> 58
   <211> 9
   <212> PRT
   <213> Homo sapiens
<400> 58
   Lys Lys Phe Ala Arg Ser Asp Glu Leu
   1 5
<210> 59
   <211> 9
   <212> PRT
   <213> Homo sapiens
<400> 59
   Ser Leu Gly Gly Pro Ala Pro Pro Pro
   1 5
<210> 60
   <211> 9
   <212> PRT
   <213> Homo sapiens
<400> 60
   Thr Leu Val Arg Ser Ala Ser Glu Thr
   1 5
<210> 61
   <211> 9
   <212> PRT
   <213> Homo sapiens
<400> 61